(19) Europäisches Patentamt / European Patent Office / Office européen des brevets

(11) EP 3 831 359 A1

(12) EUROPEAN PATENT APPLICATION
published in accordance with Art. 153(4) EPC

(43) Date of publication:
09.06.2021 Bulletin 2021/23

(51) Int Cl.:
A61K 8/37 (2006.01)    A61K 8/02 (2006.01)
A61K 8/19 (2006.01)    A61K 8/34 (2006.01)
A61K 8/55 (2006.01)    A61Q 19/00 (2006.01)

(21) Application number: 19843821.0

(22) Date of filing: 31.07.2019

(86) International application number:
PCT/JP2019/029951

(87) International publication number:
WO 2020/027177 (06.02.2020 Gazette 2020/06)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
KH MA MD TN

(30) Priority: 31.07.2018 JP 2018144602

(71) Applicant: Kao Corporation
Chuo-ku,
Tokyo 103-8210 (JP)

(72) Inventor: IWANE, Hirona
Tokyo 131-8501 (JP)

(74) Representative: Hoffmann Eitle
Patent- und Rechtsanwälte PartmbB
Arabellastraße 30
81925 München (DE)

(54) AEROSOL TYPE COSMETIC

(57) An aerosol cosmetic contains: an undiluted solution containing the following components (A), (B), (C), (D), (E), and (F): (A) an ester oil composed of a fatty acid having from 6 to 20 carbon atoms and an alcohol having from 3 to 5 carbon atoms, (B) diglycerol, (C) an anionic surfactant, (D) a nonionic surfactant having an HLB of from 3 to 20, (E) a water-soluble thickener, and (F) water, wherein the content of the component (B) is from 0.5 to 15 mass%, and the undiluted solution has a viscosity at 25°C of from 1,500 to 20,000 mPa·s; and (G) carbon dioxide gas.

EP 3 831 359 A1

## Description

Field of the Invention

The present invention relates to an aerosol cosmetic.

Background of the Invention

Skin problems such as dark circles and dullness under eyes are thought to be caused by hypoperfusion, and in order to improve them, cosmetics utilizing carbon dioxide gas effects have been studied. In particular, foam-type cosmetics which are discharged in a foam form are easy to smoothly spread and are useful for promoting the penetration of various components contained in the cosmetics by being applied to skins. For example, Patent Literature 1 describes that an aerosol-type cosmetic containing an alkyl acrylate cross polymer, a nonionic surfactant having an HLB of from 12 to 18, an oil, and a carbon dioxide gas generates a large amount of carbon dioxide gas foam having a small diameter to promote the percutaneous absorption and provide excellent feeling of use without stickiness or roughness. In addition, Patent Literature 2 describes that an aerosol cosmetic containing specific two surfactants, an acrylic acid-based polymer, a polyol, and carbon dioxide gas generates a large amount of foam having a small diameter to improve the feeling of use without stickiness, etc.

Citation List

Patent Literature

[Patent Literature 1] JP-A-2014-129306
[Patent Literature 2] JP-A-2016-17052

Summary of the Invention

The present invention relates to an aerosol cosmetic comprising:
an undiluted solution comprising the following components (A), (B), (C), (D), (E), and (F):

(A) an ester oil composed of a fatty acid having from 6 to 20 carbon atoms and an alcohol having from 3 to 5 carbon atoms,
(B) diglycerol,
(C) an anionic surfactant,
(D) a nonionic surfactant having an HLB of from 3 to 20,
(E) a water-soluble thickener, and
(F) water,
wherein the content of the component (B) is from 0.5 to 15 mass%, and the undiluted solution has a viscosity at 25°C of from 1,500 to 20,000 mPa·s; and
(G) carbon dioxide gas.

Brief Description of the Drawings

[Figure 1] Figure 1 is a diagram showing a method for measuring the concentration of carbon dioxide gas in an aerosol cosmetic in Examples.
[Figure 2] Figure 2 is a diagram showing a method for measuring the water content of keratin in Examples.

Detailed Description of the Invention

The known carbon dioxide gas-containing aerosol cosmetics described in Patent Literatures 1 and 2 still have room for improvements in providing gloss (glossy feeling) and moisturizing effect to skin after application by dissolving a high concentration of carbon dioxide gas in the cosmetic and not deteriorating the appearance and the firmness of the discharged foam.

The present inventors found that an aerosol cosmetic that solved the above problems can be obtained by combining a specific ester oil and polyol and using a specific surfactant, a water-soluble thickener, and carbon dioxide gas.

In the aerosol cosmetic of the present invention, the concentration of carbon dioxide gas (carbon dioxide) contained in the foam discharged from the container is high, the skin after application has gloss (glossy feeling), and the moisturizing effect is excellent.

The component (A) used in the present invention is an ester oil composed of a fatty acid having from 6 to 20 carbon atoms and an alcohol having from 3 to 5 carbon atoms.

The fatty acid is preferably a linear or branched fatty acid having from 8 to 18 carbon atoms.

The ester oil as the component (A) includes monoesters, diesters, and triesters composed of such a fatty acid having from 6 to 20 carbon atoms and an alcohol having from 3 to 5 carbon atoms. Among these esters, preferred are a monoester composed of a fatty acid having from 8 to 16 carbon atoms and an alcohol having from 3 to 5 carbon atoms and a diester or triester composed of a fatty acid having from 8 to 14 carbon atoms and an alcohol having from 3 to 5 carbon atoms, and the diester or triester is preferable from the viewpoint of the amount of dissolution of carbon dioxide gas, moisturizing effect, and glossy feeling.

Preferable examples of the ester oil include diester oils, such as neopentyl glycol dicaprate, propanediol dicaprylate/dicaprate, and neopentyl glycol diethylhexanoate; and triester oils, such as glyceryl tricaprylate/tricaprate and triglyceryl 2-ethylhexanoate.

The component (A) may be one ester oil or a combination of two or more ester oils, and the content thereof in the undiluted solution is preferably 0.5 mass% or more, more preferably 1 mass% or more, and further preferably 2 mass% or more from the viewpoint of the amount of dissolution of carbon dioxide gas, moisturizing effect, and glossy feeling and is preferably 20 mass% or less, more preferably 15 mass% or less, and further preferably 10 mass% or less from the viewpoint of moisturizing effect, glossy feeling, the specific volume of foam, and emulsion stability. In addition, the content of the component (A) in the undiluted solution is preferably from 0.5 to 20 mass%, more preferably from 1 to 15 mass%, and further preferably from 2 to 10 mass%.

The component (B) is diglycerol.

The content of the diglycerol as the component (B) in the undiluted solution is 0.5 mass% or more, preferably 1 mass% or more, more preferably 1.5 mass% or more, and further preferably 2.5 mass% or more from the viewpoint of glossy feeling and is 15 mass% or less, preferably 10 mass% or less, more preferably 8 mass% or less, and further preferably 6 mass% or less from the viewpoint of moisturizing effect. In addition, the content of the diglycerol in the undiluted solution is from 0.5 to 15 mass%, preferably from 1 to 10 mass%, more preferably from 1.5 to 8 mass%, and further preferably from 2.5 to 6 mass% from the viewpoint of gloss, moisture retention, and the concentration of carbon dioxide gas.

The undiluted solution of the aerosol cosmetic of the present invention can contain a polyol other than the component (B). The polyol other than the component (B) is preferably a divalent or trivalent alcohol that is used in common cosmetics.

Examples of the divalent alcohol include ethylene glycol, propylene glycol, propanediol, 1,3-butylene glycol, 1,3-propanediol, diethylene glycol, polyethylene glycol, dipropylene glycol, and polypropylene glycol; and examples of the trivalent alcohol include glycerol.

The content of the total polyols including the diglycerol as the component (B) in the undiluted solution is preferably 1 mass% or more, more preferably 2 mass% or more, and further preferably 2.5 mass% or more from the viewpoint of glossy feeling, moisturizing effect, and the specific volume of foam and is preferably 20 mass% or less, more preferably 18 mass% or less, and further preferably 15 mass% or less from the same viewpoint. In addition, the content of the total polyols including the diglycerol as the component (B) in the undiluted solution is preferably from 1 to 20 mass%, more preferably from 2 to 18 mass%, and further preferably from 2.5 to 15 mass%.

Furthermore, the content percentage (mass%) of the diglycerol in the total polyols is preferably from 20 to 100 mass%, more preferably from 25 to 80 mass%, and further preferably from 25 to 45 mass% from the viewpoint of glossy feeling, moisturizing effect, the concentration of carbon dioxide gas, and the specific volume of foam.

The anionic surfactant as the component (C) may be one used in common cosmetics, and examples thereof include alkylsulfuric acid esters having from 12 to 22 carbon atoms or salts thereof, such as sodium laurylsulfate and potassium laurylsulfate; polyoxyethylene alkyl ether phosphoric acids having from 12 to 22 carbon atoms or salts thereof, such as sodium polyoxyethylene oleyl ether phosphate, sodium polyoxyethylene stearyl ether phosphate, and sodium polyoxyethylene lauryl ether phosphate;

dialkylsulfosuccinic acids having from 12 to 24 carbon atoms or salts thereof, such as sodium di-2-ethylhexylsulfosuccinate; N-alkyloylmethyl taurines having from 12 to 22 carbon atoms or salts thereof, such as N-stearoyl-N-methyl taurine sodium; and N-$C_{12-22}$-acyl-glutamic acids or salts thereof, such as sodium dilauroyl glutamate, monosodium N-lauroyl glutamate, sodium N-stearoyl-L-glutamate, arginine N-stearoyl-L-glutamate, sodium N-stearoyl glutamate, and sodium N-myristoyl-L-glutamate. Among these surfactants, polyoxyethylene alkyl ether phosphoric acids or salts thereof and alkyloylmethyl taurines or salts thereof are preferable from the viewpoint of foam quality.

Examples of the salts thereof include alkali metals, such as sodium and potassium; alkaline earth metals, such as calcium and magnesium; ammonium; organic ammoniums derived from alkanolamines, such as monoethanolamine, diethanolamine, and triethanolamine; tris(hydroxymethyl)aminomethane; and basic amino acids, such as L-arginine.

The component (C) may be one anionic surfactant or a combination of two or more anionic surfactants, and the content thereof in the undiluted solution is preferably 0.01 mass% or more, more preferably 0.02 mass% or more, and further preferably 0.05 mass% or more and is preferably 2 mass% or less, more preferably 1.5 mass% or less, and further preferably 1 mass% or less from the viewpoint of the amount of dissolution of carbon dioxide gas, the specific volume of foam, and the emulsion stability of the ester oil as the component (A). In addition, the content of the component (C) in the undiluted solution is preferably from 0.01 to 2 mass%, more preferably from 0.02 to 1.5 mass%, and further preferably from 0.05 to 1 mass%.

The component (D) is a nonionic surfactant having an HLB of from 3 to 20.

Here, "HLB" (Hydrophilic-Lipophilic Balance) indicates the molecular weight of the hydrophilic group portion in the total molecular weight of the surfactant and is determined by Griffin's equation for nonionic surfactants.

The HLB of a surfactant mixture composed of two or more nonionic surfactants is determined as follows. The HLB of the surfactant mixture is the value obtained by averaging the HLB values of the respective nonionic surfactants based on the blending ratios thereof.

$$\texttt{Mixture HLB = } \Sigma \texttt{(HLBx} \times \texttt{Wx)/} \Sigma \texttt{Wx}$$

wherein HLBx represents the HLB value of a nonionic surfactant X; and
Wx represents the mass (g) of a nonionic surfactant X having a value of HLBx.

The nonionic surfactant as the component (D) preferably contains one or more selected from nonionic surfactants (D1) having an HLB of from 12 to 18.

Examples of the nonionic surfactant (D1) having an HLB of from 12 to 18 include polyglycerol fatty acid esters, polyethylene glycol fatty acid esters, sucrose fatty acid esters, polyoxyethylene fatty acid esters, polyoxyethylene sorbitan fatty acid esters, polyoxyethylene alkyl ethers, polyoxyethylene sorbitol fatty acid esters, polyoxyethylene glycerol fatty acid esters, polyoxyethylene propylene glycol fatty acid esters, polyoxyethylene castor oils, polyoxyethylene hydrogenated castor oils, polyoxyethylene hydrogenated castor oil fatty acid esters, polyoxyethylene phytostanol ethers, polyoxyethylene phytosterol ethers, polyoxyethylene cholestanol ethers, polyoxyethylene cholesteryl ethers, polyoxyalkylene-modified organopolysiloxanes, and polyoxyalkylene/alkyl-comodified organopolysiloxanes, and one or more selected from these nonionic surfactants can be used.

Among these nonionic surfactants, preferred are polyoxyethylene alkyl ethers and polyoxyethylene hydrogenated castor oils from the viewpoint of the foaming property at the time of discharging carbon dioxide gas.

In addition, the nonionic surfactant as the component (D) may contain a nonionic surfactant (D2) having an HLB of from 2 to 7. Examples of the nonionic surfactant (D2) having an HLB of from 2 to 7 include sorbitan fatty acid esters having from 8 to 22 carbon atoms, such as sorbitan monopalmitate, sorbitan monostearate, sorbitan monooleate, sorbitan sesquioleate, sorbitan coconut oil fatty acid, and sorbitan tristearate; alkyl glyceryl ethers, such as isostearyl glyceryl ether; and polyoxyalkylene-modified silicone, such as a polyoxyethylene/methylpolysiloxane copolymer and a poly(oxyethylene/oxypropylene)/methylpolysiloxane copolymer.

A combination of the nonionic surfactant (D1) having an HLB of from 12 to 18 and the nonionic surfactant (D2) having an HLB of from 2 to 7 can also be used.

The component (D) may be one nonionic surfactant or a combination of two or more nonionic surfactants, and the content thereof in the undiluted solution is preferably 0.05 mass% or more, more preferably 0.1 mass% or more, and further preferably 0.2 mass% or more and is preferably 5 mass% or less, more preferably 3 mass% or less, and further preferably 2 mass% or less from the viewpoint of the amount of dissolution of carbon dioxide gas, the specific volume of foam, and emulsion stability. In addition, the content of the component (D) in the undiluted solution is preferably from 0.05 to 5 mass%, more preferably from 0.1 to 3 mass%, and further preferably from 0.2 to 2 mass%.

In addition, from the viewpoint of the specific volume of foam and obtaining fine foam, the mass ratio of the nonionic surfactant as the component (D) to the anionic surfactant as the component (C), (D)/(C), is preferably 1 or more, more preferably 1.5 or more, and further preferably 2 or more and is preferably 100 or less, more preferably 50 or less, and further preferably 15 or less. In addition, the mass ratio of the component (D) to the component (C), (D)/(C), is preferably from 1 to 100, more preferably from 1.5 to 50, and further preferably from 2 to 15.

The water-soluble thickener as the component (E) may be any thickener that is used in common cosmetics, and examples thereof include carrageenan, dextrin, methyl cellulose, ethyl cellulose, hydroxyethyl cellulose, hydroxypropyl cellulose, polyvinyl alcohol, polyacrylic acid, polymethacrylic acid, a carboxyvinyl polymer, an (acrylic acid/(C10-30)alkyl acrylate) copolymer, xanthan gum, carboxymethyl chitin, and chitosan. Among these thickeners, preferred are acrylic acid-based polymers, more preferred are carboxyvinyl polymers and acrylic acid/alkyl acrylate copolymers, and more preferred are (acrylic acid/(C10-30)alkyl acrylate) copolymers, from the viewpoint of improving the water content of the horny layer, suppressing sudden generation of foam at the time of discharging, increasing the specific volume of foam, and the emulsion stability of the ester oil and the diglycerol.

Here, the (acrylic acid/(C10-30)alkyl acrylate) copolymer is a copolymer of a C10-30 alkyl acrylate and acrylic acid or methacrylic acid or a lower alkyl ester thereof and is crosslinked with an allyl ether of sucrose or an allyl ether of pentaerythritol, and a commercial product, such as Pemulen TR-1, Pemulen TR-2, Carbopol ETD2020, Carbopol 1342, and Carbopol 1382 (which are manufactured by Lubrizol Advanced Materials, Inc.), can be used.

In addition, it is preferable that acidic water-soluble thickeners, such as polyacrylic acid, a carboxyvinyl polymer, and an (acrylic acid/(C10-30)alkyl acrylate) copolymer, are used as water-soluble or water-dispersible salts by using an alkali metal hydroxide, such as potassium hydroxide and sodium hydroxide, as a neutralizer.

The component (E) may be one thickener or two or more thickeners, and the content thereof in the undiluted solution is preferably 0.1 mass% or more, more preferably 0.15 mass% or more, and further preferably 0.2 mass% or more and is preferably 1 mass% or less, more preferably 0.8 mass% or less, and further preferably 0.7 mass% or less from the viewpoint of moisture retention, glossy feeling, foam quality, and ease of application. In addition, the content of the component (E) in the undiluted solution is preferably from 0.1 to 1 mass%, more preferably from 0.15 to 0.8 mass%, and further preferably from 0.2 to 0.7 mass%.

The water as the component (F) used in the present invention functions as a solvent of the undiluted solution and balances the other components. The content of water as the component (F) in the undiluted solution is preferably 55 mass% or more, more preferably 65 mass% or more, and further preferably 75 mass% or more and is preferably 99 mass% or less, more preferably 95 mass% or less, and further preferably 90 mass% or less, from the viewpoint of moisture retention, glossy feeling, foam quality, and the emulsion stability of the undiluted solution. In addition, the content of water in the undiluted solution is preferably from 55 to 99 mass%, more preferably from 65 to 95 mass%, and further preferably from 75 to 90 mass%.

In the aerosol cosmetic of the present invention, the undiluted solution can further contain components that are used in common cosmetics, for example, an oil other than the component (A), a surfactant other than the components (C) and (D), ethanol, a preservative, an antioxidant, a pigment, a pH adjuster, a flavoring agent, an UV absorber, a moisturizing agent other than the component (B), a blood circulation promoter, a cooling sensation agent, an antiperspirant, a bactericidal agent, a whitening agent, an anti-inflammatory agent, and a skin activator.

In the aerosol cosmetic of the present invention, the undiluted solution is prepared by mixing the components (A) to (F) and other components. The undiluted solution has a viscosity at 25°C of from 1,500 to 20,000 mPa·s, preferably from 2,000 to 10,000 mPa·s, and more preferably from 2,200 to 7,000 mPa·s from the viewpoint of the emulsion stability of the undiluted solution and the discharging property of foam.

Here, the viscosity is a value measured using a BM viscometer (manufactured by Toki Sangyo Co., Ltd.) for 1 minute with a rotor No. 3 for an undiluted solution having a viscosity of less than 4,000 mPa·s and with a rotor No. 4 for an undiluted solution having a viscosity of 4,000 mPa·s or more.

An aerosol containing carbon dioxide gas does not show a blood circulation-promoting effect on the alkaline side because the gas becomes a carbonate rather than carbon dioxide. In the present invention, the undiluted solution before being filled with a propellant containing carbon dioxide gas preferably has a pH of from 4 to 7 and more preferably from 4.5 to 6.8 at 25°C from the viewpoint of dissolving carbon dioxide gas in a high concentration.

Here, the pH is a value measured at 25°C using a pH meter (F-52, manufactured by HORIBA, Ltd.).

The aerosol cosmetic of the present invention can be manufactured by filling a pressure-resistant container with the undiluted solution above and (G) carbon dioxide gas. The injection manner is preferably a foam type to discharge in a foam form.

The mass ratio of the undiluted solution and the carbon dioxide gas is preferably from 94:6 to 99.5:0.5, more preferably from 95:5 to 99:1, and further preferably from 96.5:3.5 to 98.5:1.5 from the viewpoint of the solubility of carbon dioxide gas in the undiluted solution and foam generation.

The aerosol cosmetic of the present invention may contain a propellant that is used in common aerosol cosmetics, in addition to the carbon dioxide gas as the component (G). Examples of the propellant include liquefied petroleum gas and compressed gas. When a propellant other than carbon dioxide gas is contained, the content of carbon dioxide gas in the total amount of the carbon dioxide gas and the propellant is preferably 90 mass% or more, more preferably 95 mass% or more, and further preferably 98 mass% or more.

Although the aerosol cosmetic of the present invention can be applied as a cosmetic without specific limitation, since carbon dioxide gas is percutaneously absorbed to give a blood circulation-promoting effect higher than ever before, it is suitable as a skin cosmetic that is used for, for example, improving the skin color (such as brightness and dullness) or improving the moisturizing feeling.

When the aerosol cosmetic of the present invention is applied to skin and then left to stand for a certain time, since a state that a liniment containing foam has formed a kind of coating film is obtained, the effect of promoting blood circulation can be enhanced. Furthermore, the active component in the cosmetic can also be simultaneously penetrated, which is considered to be very beneficial.

The aerosol cosmetic of the present invention can be used by, for example, applying to skin, leaving to stand for a certain time, spreading on skin, wiping off, or washing away, depending on the usage situation.

The aerosol cosmetic of the present invention can be manufactured by a common method and, for example, can be manufactured by the following steps 1 to 4:

Step 1: uniformly mixing the components (E) and (F) and a basic component,
(in this step 1, it is preferable to heat to 50°C or more);
Step 2: adding the components (A), (B), (C), and (D) and optionally other components to the liquid prepared in step 1, followed by uniformly stirring the mixture;
Step 3: cooling the liquid prepared in step 2 to from 15°C to 30°C; and
Step 4: filling the liquid prepared in step 3 (also referred to as an undiluted solution) into a pressure-resistant container and then sealing it, followed by filling the component (G) by pressuring.

Regarding the above-described embodiments, the present invention further discloses the following compositions:

<1> An aerosol cosmetic comprising:
an undiluted solution comprising the following components (A), (B), (C), (D), (E), and (F):

(A) an ester oil composed of a fatty acid having from 6 to 20 carbon atoms and an alcohol having from 3 to 5 carbon atoms,
(B) diglycerol,
(C) an anionic surfactant,
(D) a nonionic surfactant having an HLB of from 3 to 20,
(E) a water-soluble thickener, and
(F) water,
wherein the content of the component (B) is from 0.5 to 15 mass%, and the undiluted solution has a viscosity at 25°C of from 1,500 to 20,000 mPa·s; and
(G) carbon dioxide gas;

<2> The aerosol cosmetic according to aspect <1>, wherein the component (A) is preferably a monoester composed of a fatty acid having from 8 to 16 carbon atoms and an alcohol having from 3 to 5 carbon atoms or a diester or triester composed of a fatty acid having from 8 to 14 carbon atoms and an alcohol having from 3 to 5 carbon atoms and is more preferably the diester or triester;

<3> The aerosol cosmetic according to aspect <1> or <2>, wherein the content of the component (A) in the undiluted solution is preferably 0.5 mass% or more, more preferably 1 mass% or more, and further preferably 2 mass% or more and is preferably 20 mass% or less, more preferably 15 mass% or less, and further preferably 10 mass% or less;

<4> The aerosol cosmetic according to any one of aspects <1> to <3>, wherein the content of the diglycerol as the component (B) in the undiluted solution is preferably 1 mass% or more, more preferably 1.5 mass% or more, and further preferably 2.5 mass% or more and is preferably 10 mass% or less, more preferably 8 mass% or less, and further preferably 6 mass% or less;

<5> The aerosol cosmetic according to any one of aspects <1> to <4> can further include a polyol other than the component (B) and preferably contains a divalent or trivalent alcohol;

<6> The aerosol cosmetic according to any one of aspects <1> to <5>, wherein the content of the total polyols including the diglycerol as the component (B) in the undiluted solution is preferably 1 mass% or more, more preferably 2 mass% or more, and further preferably 2.5 mass% or more and is preferably 20 mass% or less, more preferably 18 mass% or less, and further preferably 15 mass% or less;

<7> The aerosol cosmetic according to any one of aspects <1> to <6>, wherein the anionic surfactant as the component (C) is preferably a polyoxyethylene alkyl ether phosphoric acid or a salt thereof or an alkyloylmethyl taurine or a salt thereof;

<8> The aerosol cosmetic according to any one of aspects <1> to <7>, wherein the content of the component (C) in the undiluted solution is preferably 0.01 mass% or more, more preferably 0.02 mass% or more, and further preferably 0.05 mass% or more and is preferably 2 mass% or less, more preferably 1.5 mass% or less, and further preferably 1 mass% or less;

<9> The aerosol cosmetic according to any one of aspects <1> to <8>, wherein the nonionic surfactant as the component (D) preferably comprises at least one selected from nonionic surfactants (D1) having an HLB of from 12 to 18, and is more preferably a polyoxyethylene alkyl ether or a polyoxyethylene hydrogenated castor oil;

<10> The aerosol cosmetic according to any one of aspects <1> to <9>, wherein the nonionic surfactant as the component (D) preferably comprises a nonionic surfactant (D2) having an HLB of from 2 to 7, and is more preferably a sorbitan fatty acid ester having from 8 to 22 carbon atoms, an alkyl glyceryl ether, or polyoxyalkylene-modified silicone;

<11> The aerosol cosmetic according to any one of aspects <1> to <10>, wherein the nonionic surfactant as the component (D) preferably comprises a nonionic surfactant (D1) having an HLB of from 12 to 18 and a nonionic surfactant (D2) having an HLB of from 2 to 7;

<12> The aerosol cosmetic according to any one of aspects <1> to <11>, wherein the content of the component (D) in the undiluted solution is preferably 0.05 mass% or more, more preferably 0.1 mass% or more, and further preferably 0.2 mass% or more and is preferably 5 mass% or less, more preferably 3 mass% or less, and further preferably 2 mass% or less;

<13> The aerosol cosmetic according to any one of aspects <1> to <12>, wherein the mass ratio of the nonionic surfactant as the component (D) to the anionic surfactant as the component (C), (D)/(C), is preferably 1 or more, more preferably 1.5 or more, and further preferably 2 or more and is preferably 100 or less, more preferably 50 or less, and further preferably 15 or less;

<14> The aerosol cosmetic according to any one of aspects <1> to <13>, wherein the water-soluble thickener as the component (E) is preferably an acrylic acid-based polymer, more preferably a carboxyvinyl polymer or an acrylic acid/alkyl acrylate copolymer, and more preferably an (acrylic acid/(C10-30)alkyl acrylate) copolymer;

<15> The aerosol cosmetic according to any one of aspects <1> to <14>, wherein the content of the component (E) in the undiluted solution is preferably 0.1 mass% or more, more preferably 0.15 mass% or more, and further preferably 0.2 mass% or more and is preferably 1 mass% or less, more preferably 0.8 mass% or less, and further preferably 0.7 mass% or less;

<16> The aerosol cosmetic according to any one of aspects <1> to <15>, wherein the content of water as the component (F) in the undiluted solution is preferably 55 mass% or more, more preferably 65 mass% or more, and further preferably 75 mass% or more and is preferably 99 mass% or less, more preferably 95 mass% or less, and further preferably 90 mass% or less;

<17> The aerosol cosmetic according to any one of aspects <1> to <16>, wherein the undiluted solution preferably has a viscosity at 25°C of from 2,000 to 10,000 mPa·s, more preferably from 2,200 to 7,000 mPa-s;

<18> The aerosol cosmetic according to any one of aspects <1> to <17>, wherein the undiluted solution before being filled with a propellant containing carbon dioxide gas preferably has a pH of from 4 to 7, more preferably from 4.5 to 6.8, at 25°C;

<19> The aerosol cosmetic according to any one of aspects <1> to <18>, wherein the mass ratio of the undiluted solution and the carbon dioxide gas is preferably from 94:6 to 99.5:0.5, more preferably from 95:5 to 99:1, and further preferably from 96.5:3.5 to 98.5:1.5;

<20> The aerosol cosmetic according to any one of aspects <1> to <19> being a skin cosmetic that is used for, for example, improving the skin color (such as brightness and dullness) or improving the moisturizing feeling;

<21> The aerosol cosmetic according to any one of aspects <1> to <20> being used by applying to skin, leaving to stand for a certain time, spreading on skin, wiping off, or washing away;

<22> The aerosol cosmetic according to any one of aspects <1> to <21>, wherein the content percentage (mass%) of the diglycerol in the total polyols is preferably from 20 to 100 mass%, more preferably from 25 to 80 mass%, and further preferably from 25 to 45 mass%; and

<23> The aerosol cosmetic according to any one of aspects <1> to <22> capable of being manufactured by the following steps 1 to 4:

Step 1: uniformly mixing the components (E) and (F) and a basic component,
(in this step 1, it is preferable to heat to 50°C or more);
Step 2: adding the components (A), (B), (C), and (D) and optionally other components to the liquid prepared in step 1, followed by uniformly stirring;
Step 3: cooling the liquid prepared in step 2 to from 15°C to 30°C; and
Step 4: filling a pressure-resistant container with the liquid prepared in step 3 (also referred to as an undiluted solution) and sealing it, followed by filling with the component (G) by pressuring.

Examples

A method for manufacturing aerosol cosmetics and a method for evaluating them in the following Examples were as follows.

(Manufacturing method)

Step 1: Components (E) and (F) and a basic component were uniformly mixed at a temperature of from 60°C to 70°C;
Step 2: Components (A), (B), (C), (D), and (G) and other components were added to the solution prepared in step 1, followed by uniformly stirring;
Step 3: The solution prepared in step 2 was cooled to 15°C to 30°C; and
Step 4: A pressure-resistant container was filled with the solution (undiluted solution) prepared in step 3 and was sealed and then filled with component (G) by pressuring to obtain an aerosol cosmetic.

(Evaluation method)

(1) Viscosity of undiluted solution:
A 50-mL glass container was filled with 50 g of the undiluted solution, and measurement with a BM viscometer (manufactured by Toki Sangyo Co., Ltd.) was performed (rotor No. 4, 12 rpm, 1 min).
(2) pH of undiluted solution:
The pH of the undiluted solution was measured with a pH meter (F-52, manufactured by HORIBA, Ltd.) at 25°C immediately after manufacturing. The pH was measured directly without diluting the undiluted solution at the time of measuring.
(3) Concentration of carbon dioxide gas:
The solubility of carbon dioxide gas in each cosmetic was calculated from the pressure inside the can of the aerosol cosmetic at 12 hours after the filling with carbon dioxide gas by the following procedure:

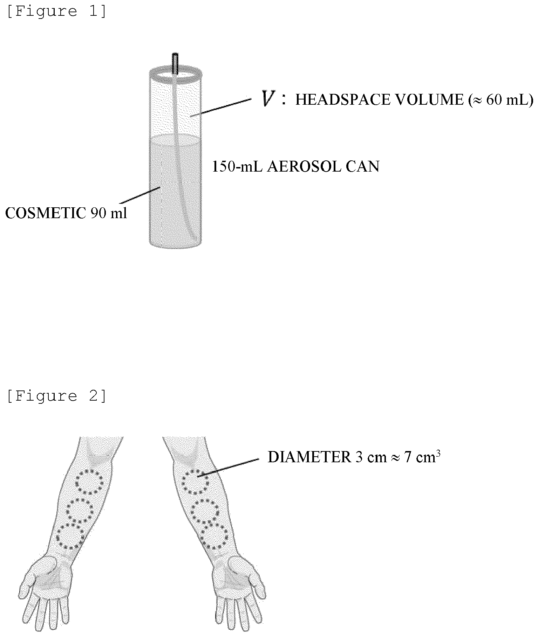

(a) The pressure inside the can of each aerosol cosmetic was measured using a strain gauge converter;
(b) The weight of carbon dioxide gas in the headspace was calculated from the headspace volume (about 60 mL, Figure 1) and the pressure inside the can of the aerosol cosmetic using the van der Waals equation of state;
(c) The weight of carbon dioxide gas dissolved in the cosmetic was calculated by subtracting the calculated weight of carbon dioxide gas in the headspace from the weight of the filled carbon dioxide gas; and
(d) The weight of carbon dioxide gas dissolved in the cosmetic was further converted to the value under atmospheric pressure (1 atm) according to Henry's law to obtain the concentration of carbon dioxide gas of the cosmetic.

<Van der Waals equation of state>

$$\left( P + \frac{n^2 a}{V^2} \right) (V - nb) = nRT$$

P: carbon dioxide gas equilibrium pressure (25°C)
n: molar mass of carbon dioxide gas in headspace

R = 0.0821 L atm/Kmol, T = 298 L, V = 0.004 L
a = 3.65 atm $L^2/mol^2$, b = 0.0428 L/mol

(4) Water content of keratin:
An aerosol cosmetic 12 hours after the filling with carbon dioxide gas was applied to skin, and the water content of keratin was evaluated according to the following procedure:

(a) A formulation was prepared by discharging an aerosol cosmetic into a 50-mL sample bottle and leaving it to stand for 10 minutes, and 0.05 mL of the formulation was applied to the central part of each test site (a circle of 3 cm in diameter) of the inner forearm shown in Figure 2 with a 1-mL syringe;
(b) The discharged sample was spread to the entire skin within the circle with a fingertip; and
(c) At 15 minutes after the spread to the skin, the water content of the horny layer (arbitrary unit; au) was measured with Corneometer CM 825 (manufactured by Courage + Khazaka electronic GmbH, Germany), and the difference (Δ value) from that when not applied as a control was determined.

(5) Skin gloss value and gloss:

An aerosol cosmetic was applied to each test site by the same method as that in the paragraph (4). At 5 minutes after the spread to the skin, the skin gloss value (arbitrary unit; au) was measured with Glossymeter GL 200 (manufactured by Courage + Khazaka electronic GmbH, Germany), and the difference (Δ value) from that when not applied as a control was used for evaluation of gloss according to the following criteria:

d: less than 2.0,
c: 2.0 or more and less than 2.5,
b: 2.5 or more and less than 2.75, and
a: 2.75 or more.

(6) Specific volume of foam:

Foam was discharged into a 10-mL petri dish, the weight of the foam precisely filing the petri dish was measured, and the specific volume of the foam ($cm^3/g$) was calculated.

Examples 1 to 10 and Comparative Examples 1 to 5

Aerosol cosmetics having compositions shown in Table 1 were manufactured. The viscosity and the pH at 25°C of each undiluted solution were measured, and the water content of the horny layer, the skin gloss value, and the glossy feeling were also evaluated. The results are collectively shown in Table 1.

[Table 1]

| Component (mass%) | | Example 1 | Example 2 | Example 3 | Example 4 | Example 5 | Example 6 | Example 7 | Example 8 | Example 9 | Example 10 | Comparative Example 1 | Comparative Example 2 | Comparative Example 3 | Comparative Example 4 | Comparative Example 5 |
|---|---|---|---|---|---|---|---|---|---|---|---|---|---|---|---|---|
| (A) | Propanediol dicaprylate/dicaprate (SALACOS PR-85, manufactured by The Nisshin Oillio Group, Ltd.) | | | | | | | 2.5 | | | | | | | 2.5 | |
| | Neopentyl glycol diethylhexanoate (COSMOL 525, manufactured by The Nisshin Oillio Group, Ltd.) | | | | | | 2.5 | | | | | | | | | |
| | Neopentyl glycol dicaprate (ESTEMOL N-01, manufactured by The Nisshin Oillio Group, Ltd.) | 4.5 | 4.5 | 3.5 | 20 | | 2 | 2 | 4.5 | 4.5 | 4.5 | 0 | | | 2 | 4.5 |
| | Glyceryl tricaprylate/tricaprate (COCONAD MT, manufactured by Kao Corporation) | | | | | 4.5 | | | | | | | | | | |

EP 3 831 359 A1

| Component (mass%) | | Example 1 | Example 2 | Example 3 | Example 4 | Example 5 | Example 6 | Example 7 | Example 8 | Example 9 | Example 10 | Comparative Example 1 | Comparative Example 2 | Comparative Example 3 | Comparative Example 4 | Comparative Example 5 |
|---|---|---|---|---|---|---|---|---|---|---|---|---|---|---|---|---|
| | Isostearyl isostearate (ISIS, manufactured by Kokyu Alcohol Kogyo Co., Ltd.) | | | | | | | | | | | | | 4.5 | | |
| | Squalane | | | | | | | | | | | | 4.5 | | | |
| (B) | Diglycerol | 3 | 3 | 3 | 3 | 3 | 3 | 3 | 2 | 5 | 7 | 3 | 3 | 3 | 0 | 0 |
| | Dipropylene glycol | | 5 | 5 | 5 | 5 | 5 | 5 | 5 | 5 | 5 | 5 | 5 | 5 | 5 | 5 |
| | Propanediol | | 2 | 2 | 2 | 2 | 2 | 2 | 2 | 2 | 2 | 2 | 2 | 2 | 2 | 2 |
| | Glycerol | | | | | | | | | | | | | | | 3 |
| (C) | Sodium polyoxyethylene lauryl ether phosphate (SPE-104NB, manufactured by Kao Corporation) | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |

EP 3 831 359 A1

11

| Component (mass%) | | Example 1 | Example 2 | Example 3 | Example 4 | Example 5 | Example 6 | Example 7 | Example 8 | Example 9 | Example 10 | Comparative Example 1 | Comparative Example 2 | Comparative Example 3 | Comparative Example 4 | Comparative Example 5 |
|---|---|---|---|---|---|---|---|---|---|---|---|---|---|---|---|---|
| (D) | Polyoxyethylene hydrogenated castor oil (Emanon CH60K, manufactured by Kao Corporation) | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| | Isostearyl glyceryl ether (Penetol GE-IS, manufactured by Kao Corporation) | 0.1 | | 0.1 | 0.1 | 0.1 | 0.1 | 0.1 | 0.1 | 0.1 | 0.1 | 0.1 | 0.1 | 0.1 | 0.1 | 0.1 |
| (E) | Acrylic acid/alkyl methacrylate copolymer (Pemulen TR-1, manufactured by Lubrizol Advanced Materials, Inc.) | 0.4 | 0.4 | 0.4 | 0.4 | 0.4 | 0.4 | 0.4 | 0.4 | 0.4 | 0.4 | 0.4 | 0.4 | 0.4 | 0.4 | 0.4 |
| | Potassium dihydrogen phosphate | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 |
| | 48% potassium hydroxide | 0.45 | 0.45 | 0.45 | 0.45 | 0.45 | 0.45 | 0.45 | 0.45 | 0.45 | 0.45 | 0.45 | 0.45 | 0.45 | 0.45 | 0.45 |
| | Phenoxy ethanol | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |

EP 3 831 359 A1

(continued)

| Component (mass%) | | Example 1 | Example 2 | Example 3 | Example 4 | Example 5 | Example 6 | Example 7 | Example 8 | Example 9 | Example 10 | Comparative Example 1 | Comparative Example 2 | Comparative Example 3 | Comparative Example 4 | Comparative Example 5 |
|---|---|---|---|---|---|---|---|---|---|---|---|---|---|---|---|---|
| | Disodium edetate | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 |
| | Flavoring agent | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 |
| (F) | Purified water | Balance | Balance | Balance | Balance | Balance | Balance | Balance | Balance | Balance | Balance | Balance | Balance | Balance | Balance | Balance |
| | Undiluted solution total | 100 | 100 | 100 | 100 | 100 | 100 | 100 | 100 | 100 | 100 | 100 | 100 | 100 | 100 | 100 |
| | Undiluted solution | 97.6 | 97.6 | 97.6 | 97.6 | 97.6 | 97.6 | 97.6 | 97.6 | 97.6 | 97.6 | 97.6 | 97.6 | 97.6 | 97.6 | 97.6 |
| (G) | Carbon dioxide gas | 2.4 | 2.4 | 2.4 | 2.4 | 2.4 | 2.4 | 2.4 | 2.4 | 2.4 | 2.4 | 2.4 | 2.4 | 2.4 | 2.4 | 2.4 |
| | Total | 100 | 100 | 100 | 100 | 100 | 100 | 100 | 100 | 100 | 100 | 100 | 100 | 100 | 100 | 100 |
| | Undiluted solution viscosity (mPa-s) | 4950 | 4630 | 4330 | 6300 | 4720 | 4920 | 4300 | 4660 | 4670 | 4250 | 5280 | 5250 | 4860 | 4630 | 4950 |
| | Undiluted solution pH | 6.6 | 6.5 | 6.6 | 6.7 | 6.5 | 6.5 | 6.5 | 6.6 | 6.6 | 6.5 | 6.5 | 6.5 | 6.637 | 6.4 | 6.6 |
| | Horny layer water content | 26.4 | 31.9 | 35 | 28.5 | 38.7 | 38.4 | 31.4 | 36.3 | 34.9 | 34.5 | 29.8 | 24.6 | 31.2 | 24.1 | 31.6 |
| | Gloss | b | a | a | a | b | a | a | b | a | a | d | d | c | c | c |
| | Skin gloss value | 2.71 | 2.85 | 3.08 | 4.19 | 2.57 | 2.91 | 2.87 | 2.51 | 2.97 | 3.29 | 1.04 | 1.94 | 2.19 | 2.14 | 2.21 |

The results of Table 1 demonstrate that all of the products of the present invention provide gloss (glossy feeling) to the skin after application and show good moisturizing effects. Comparative Examples 1 to 3 not containing the ester oil as the component (A) and Comparative Examples 4 and 5 not containing the diglycerol as the component (B) were inferior in gloss (glossy feeling).

Examples 2, 11, and 12 and Comparative Examples 6 and 7

Aerosol cosmetics having compositions shown in Table 2 were manufactured. The viscosity and the pH at 25°C of each undiluted solution were measured, and the specific volume of foam was also evaluated. The results are collectively shown in Table 2.

[Table 2]

| | Component (mass%) | Example 2 | Example 11 | Example 12 | Comparative Example 6 | Comparative Example 7 |
|---|---|---|---|---|---|---|
| (A) | Neopentyl glycol dicaprate (ESTEMOL N-01, manufactured by The Nisshin OilIio Group, Ltd.) | 4.5 | 4.5 | 4.5 | 4.5 | 4.5 |
| (B) | Diglycerol | 3 | 3 | 3 | 3 | 3 |
| | Dipropylene glycol | 5 | 5 | 5 | 5 | 5 |
| | Propanediol | 2 | 2 | 2 | 2 | 2 |
| (C) | Sodium polyoxyethylene lauryl ether phosphate (SPE-104NB, manufactured by Kao Corporation) | 0.1 | 0.05 | 0.2 | 0 | 0.1 |
| (D) | Polyoxyethylene hydrogenated castor oil (Emanon CH60K, manufactured by Kao Corporation) | 0.5 | 0.5 | 0.5 | 0.5 | 0 |
| | Isostearyl glyceryl ether (Penetol GE-IS, manufactured by Kao Corporation) | 0 | 0.1 | 0.2 | 0.1 | 0 |
| (E) | Acrylic acid/alkyl methacrylate copolymer (Pemulen TR-1, manufactured by Lubrizol Advanced Materials, Inc.) | 0.4 | 0.4 | 0.4 | 0.4 | 0.4 |
| | Potassium dihydrogen phosphate | 0.2 | 0.2 | 0.2 | 0.2 | 0.2 |
| | 48% potassium hydroxide | 0.45 | 0.45 | 0.45 | 0.45 | 0.45 |
| | Phenoxyethanol | 0.5 | 0.5 | 0.5 | 0.5 | 0.5 |
| | Disodium edetate | 0.05 | 0.05 | 0.05 | 0.05 | 0.05 |
| | Flavoring agent | 0.1 | 0.1 | 0.1 | 0.1 | 0.1 |
| (F) | Purified water | Balance | Balance | Balance | Balance | Balance |
| | Undiluted solution total | 100 | 100 | 100 | 100 | 100 |
| | Undiluted solution | 97.6 | 97.6 | 97.6 | 97.6 | 97.6 |
| (G) | Carbon dioxide gas | 2.4 | 2.4 | 2.4 | 2.4 | 2.4 |
| | Total | 100 | 100 | 100 | 100 | 100 |
| | Undiluted solution viscosity (mPa·s) | 4630 | 4640 | 4250 | 6240 | 5410 |
| | Undiluted solution pH | 6.5 | 6.7 | 6.5 | 6.3 | 6.6 |
| | Specific volume of foam ($cm^3$/g) | 4.46 | 4.34 | 4.28 | 3.95 | 4.01 |

The results of Table 2 demonstrate that Comparative Example 6 not containing the anionic surfactant as the component (C) and Comparative Example 7 not containing the nonionic surfactant as the component (D) did not provide sufficient foam.

The concentration of carbon dioxide gas and the specific volume of foam of each of the aerosol cosmetics of Examples 1 to 10 and Comparative Examples 1 and 2 were measured. The results are shown in Table 3.

In all of the products of the present invention, the foam quality was good, and the carbon dioxide gas was dissolved at a high concentration. In contrast, in Comparative Examples 1 and 2, the concentration of carbon dioxide gas was not sufficient.

[Table 3]

| | Example 1 | Example 2 | Example 3 | Example 4 | Example 5 | Example 6 | Example 7 | Example 8 | Example 9 | Example 10 | Comparative Example 1 | Comparative Example 2 |
|---|---|---|---|---|---|---|---|---|---|---|---|---|
| Carbon dioxide gas concentration (ppm) | 1670 | 1620 | 1610 | 2040 | 1625 | 1640 | 1630 | 1640 | 1615 | 1605 | 1490 | 1525 |
| Specific volume of foam ($cm^3$/g) | 4.26 | 4.46 | 4.2 | 4.12 | 4.2 | 4.28 | 4.3 | 4.3 | 4.27 | 4.24 | 4.59 | 4.86 |

Prescription example

An undiluted solution of the prescription below was prepared in the same manner as in Examples. A pressure-resistant container was filled with this undiluted solution and carbon dioxide gas at a ratio of 97.6:2.4 to prepare an aerosol cosmetic.

The resulting aerosol cosmetic has a good foam quality, contains carbon dioxide gas dissolved at a high concentration, provides gloss (glossy feeling) to the skin after application, and has an excellent moisturizing effect.

[Table 4]

| (Undiluted solution prescription) | |
|---|---|
| Propanediol dicaprylate/dicaprate | 2.5 (mass%) |
| Neopentyl glycol dicaprate | 2 |
| Diglycerol | 3 |
| Dipropylene glycol | 5 |
| 1,3-Propanediol | 2 |
| Sodium polyoxyethylene lauryl ether phosphate | 0.1 |
| Polyoxyethylene hydrogenated castor oil | 0.5 |
| Isostearyl glyceryl ether | 0.1 |
| Acrylic acid/alkyl methacrylate copolymer | 0.4 |
| Phenoxyethanol | 0.5 |
| Disodium edetate | 0.05 |
| Potassium dihydrogen phosphate | 0.2 |
| Potassium hydroxide solution (A) | 0.45 |
| Flavoring agent | 0.1 |
| Dipotassium glycyrrhizinate | 0.1 |
| Lauryl methacrylate/ethylene glycol dimethacrylate/ sodium methacrylate copolymer aqueous dispersion | 1.25 |
| 1-Menthyl glyceryl ether | 0.01 |
| Hydrolyzed silk liquid | 0.01 |
| Oenothera biennis extract | 0.01 |
| Clove extract | 0.3 |
| N-Acetylglucosamine | 0.07 |
| Dioscorea panthaica root extract | 0.05 |
| Perilla extract (1) | 0.003 |
| Purified water | Balance |
| Total | 100 |

**Claims**

1. An aerosol cosmetic comprising:
   an undiluted solution comprising the following components (A), (B), (C), (D), (E), and (F):

   (A) an ester oil composed of a fatty acid having from 6 to 20 carbon atoms and an alcohol having from 3 to 5 carbon atoms,
   (B) diglycerol,
   (C) an anionic surfactant,
   (D) a nonionic surfactant having an HLB of from 3 to 20,
   (E) a water-soluble thickener, and
   (F) water, wherein a content of the component (B) is from 0.5 to 15 mass%, and the undiluted solution has a viscosity at 25°C of from 1,500 to 20,000 mPa·s; and
   (G) carbon dioxide gas.

2. The aerosol cosmetic according to Claim 1, wherein the component (C) is comprised in the undiluted solution in an amount of from 0.01 to 2 mass%.

3. The aerosol cosmetic according to Claim 1 or 2, wherein the nonionic surfactant as the component (D) comprises a nonionic surfactant (D1) having an HLB of from 12 to 18.

4. The aerosol cosmetic according to any one of Claims 1 to 3, wherein a content of the component (A) in the undiluted solution is 0.5 mass% or more and 20 mass% or less.

5. The aerosol cosmetic according to any one of Claims 1 to 4, wherein a mass ratio of the nonionic surfactant as the component (D) to the anionic surfactant as the component (C), (D)/(C), is 1 or more and 100 or less.

6. The aerosol cosmetic according to any one of Claims 1 to 5, wherein a content percentage (mass%) of the diglycerol in the total polyols is from 20 to 100 mass%.

7. The aerosol cosmetic according to any one of Claims 1 to 6, wherein a content of the component (E) in the undiluted solution is 0.1 mass% or more and 1 mass% or less.

8. The aerosol cosmetic according to any one of Claims 1 to 7, wherein a content of the component (F) in the undiluted solution is 55 mass% or more and 99 mass% or less.

[Figure 1]

$V$ : HEADSPACE VOLUME ($\approx$ 60 mL)

150-mL AEROSOL CAN

COSMETIC 90 ml

[Figure 2]

DIAMETER 3 cm $\approx$ 7 cm$^3$

## INTERNATIONAL SEARCH REPORT

| International application No. |
|---|
| PCT/JP2019/029951 |

### A. CLASSIFICATION OF SUBJECT MATTER
Int.Cl. A61K8/37(2006.01)i, A61K8/02(2006.01)i, A61K8/19(2006.01)i, A61K8/34(2006.01)i, A61K8/55(2006.01)i, A61Q19/00(2006.01)i

According to International Patent Classification (IPC) or to both national classification and IPC

### B. FIELDS SEARCHED

Minimum documentation searched (classification system followed by classification symbols)
Int.Cl. A61K8/37, A61K8/02, A61K8/19, A61K8/34, A61K8/55, A61Q19/00

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2014-129306 A (KAO CORP.) 10 July 2014, claims 1-3, paragraphs [0023], [0029]-[0030], [0032], [0035], example 11 (Family: none) | 1-8 |
| Y | JP 2017-218441 A (NOF CORPORATION) 14 December 2017, claim 1, paragraph [0020], example 1, comparative example 2 (Family: none) | 1-8 |

☐ Further documents are listed in the continuation of Box C.  ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 17 October 2019 (17.10.2019) | 05 November 2019 (05.11.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**REFERENCES CITED IN THE DESCRIPTION**

**Patent documents cited in the description**

- JP 2014129306 A
- JP 2016017052 A